# EUROPEAN PATENT SPECIFICATION

(11) **EP 2 142 070 B1**
(45) Date of publication and mention of the grant of the patent: **06.01.2021**
(21) Application number: 08769222.4
(22) Date of filing: 28.04.2008
(51) Int. Cl.: A61M 25/04, A61B 5/042, A61M 25/00, A61B 17/00, A61B 17/22, A61B 17/34, A61B 90/00, A61B 34/10, A61B 34/20

(54) **CATHETER**
KATHETER
CATHÉTER

(30) Priority: 27.04.2007 US 914575 P
(43) Date of publication of application: 13.01.2010
(73) Proprietor: St. Jude Medical, Atrial Fibrillation Division, Inc., St. Paul, Minnesota 55117-9913 (US)
(72) Inventor: CHRISTIAN, Steven, C., New Brighton, Minnesota 55112 (US); PURYEAR, Harry, A., Shoreview, Minnesota 55126 (US); KAUPHUSMAN, James, Champlin, Minnesota 55316 (US); SUTTON, William, Minnetonka, Minnesota 55345 (US)
(74) Representative: Kramer Barske Schmidtchen Patentanwälte PartG mbB
(86) International application number: PCT/US2008/061802
(87) International publication number: WO 2008/134651

(56) References cited:
- EP-A2- 1 180 380
- WO-A1-02/19934
- US-A- 5 314 460
- US-A- 5 573 508
- US-A- 5 593 405
- US-A1- 2004 097 788
- US-A1- 2004 215 186
- US-B1- 6 267 746

## Description

### BACKGROUND

### a. Technical Field

The instant invention relates generally to the navigation of a medical device through a patient. More specifically, the instant invention relates to positioning and retaining a reference electrode of a localization system employed in navigating a medical device through a patient.

### b. Background Art

It is well known to generate heart chamber geometry in preparation for cardiac diagnostic or therapeutic procedures. Often, a mapping catheter is introduced into the heart chamber of interest and moved around within the heart chamber, either randomly, pseudo-randomly, or according to one or more preset patterns. The three-dimensional coordinates are measured using a localization system (sometimes also referred to as a "mapping system," "navigation system," or "positional feedback system"). The localization system measures the coordinates of the mapping catheter within a localization field, typically by relating a characteristic of the localization field, such as a voltage, experienced by the mapping catheter to a location of the catheter within the field. A similar process may be used to measure the position of any object, such as an ablation catheter or other medical device, within the localization field.

It is desirable for the three-dimensional coordinate system of the localization system to have a stable reference point or origin. While any stable position will suffice, it is desirable for many reasons to utilize a reference point that is proximate to the mapping catheter. Thus, a catheter-mounted reference electrode is often inserted into the heart and positioned in a fixed location, for example the coronary sinus, to establish the origin of the coordinate system relative to which the location of the mapping catheter will be measured.

It is known, however, that the stationary reference electrode may become dislodged. For example, the mapping catheter may collide or become entangled with the reference electrode, or the physician moving the mapping catheter may inadvertently jostle the catheter carrying the reference electrode. The reference electrode may also be dislodged by patient movement.

When the reference electrode becomes dislodged, it effectively shifts the origin of the coordinate system relative to which the position of the mapping catheter is measured. Unless the dislodgement is detected and accounted for, positions of the mapping catheter measured after the dislodgement will be invalid.

US 2004/0215186 A1 refers to electrical block positioning devices and discloses a guiding and anchoring catheter which have an anchoring section, for example shaped as a loop. This guiding and anchoring catheter is used to insert an ablation catheter to a predetermined location within a patient's heart. The anchoring device is not fixed in position with respect to the electrode, i.e. the ablation catheter. The anchoring catheter is not disclosed to bear an electrode.

EP 1 180 380 A2 refers to a catheter apparatus for medical treatment. This catheter comprises electrodes along its shaft portion and an expandable nitinol wire portion for anchoring.

US 5,593,405 discloses an endoscope having a conductor assembly. This conductor assembly can be used for point by point mapping or scanning of the internal surface of the heart and/or for transmission of cardiac signals and/or for determination of electrical potentials in a patient's heart. The device can be anchored by means of a lasso-shaped anchoring portion and comprises electrodes which have a fixed relation with respect to the anchoring device.

### BRIEF SUMMARY

Accordingly, it is an object of the present invention to provide devices for anchoring a reference electrode for use in a mapping procedure.

It is another object of the present invention to provide devices for anchoring a reference electrode within a vessel while preserving perfusion through the vessel.

It is still another object of the present invention to provide devices to anchor an electrode within a vessel for use in diagnostic and/or therapeutic procedures.

In a first aspect, the invention provides a catheter for anchoring an electrode in a coronary sinus. The catheter includes: an elongate catheter body adapted to be inserted into a coronary sinus, the elongate catheter body including an anchor section having an expandable axial cross-section; at least one electrode on the catheter body; and an actuation mechanism operably coupled to the anchor section to actuate the anchor section between an undeployed configuration, wherein the expandable axial cross-section of the anchor section is in a collapsed state, and a deployed configuration, wherein the expandable axial cross-section of the anchor section is in an expanded state. When the anchor section is in the deployed configuration, the expandable axial cross-section engages a tissue surface of the coronary sinus to inhibit movement between the catheter body and the coronary sinus without completely occluding the coronary sinus.

The at least one electrode may be positioned distally of the anchor section, proximally of the anchor section, and/or on the anchor section.

The catheter body includes at least one perfusion passage having a first opening positioned distally of the anchor section and a second opening positioned proximally of the anchor section. This permits perfusion through the interior of the catheter body. In addition, the catheter is configured so that perfusion occurs through the vessel around the exterior of the catheter body.

Optionally, the actuation mechanism includes a tension member. Placing the tension member in tension may cause the anchor section to assume the deployed configuration.

In some embodiments of the invention, the anchor section includes at least one expandable member mounted to an outer surface of the catheter body. The at least one expandable member may include at least one balloon or at least one wire basket.

Also disclosed herein is a catheter for anchoring an electrode in a coronary sinus, including: an elongate catheter body adapted to be inserted into a coronary sinus; at least one wire anchor coupled to the catheter body; and at least one electrode on the catheter body. The at least one wire anchor is movable between an undeployed configuration, wherein the catheter body is movable relative to the coronary sinus, and a deployed configuration, wherein the at least one wire anchor engages a tissue surface of the coronary sinus to inhibit movement between the catheter body and the coronary sinus without completely occluding the coronary sinus. Optionally, the catheter may also include at least one wire lock that couples the at least one wire anchor to the catheter body.

In some examples, the catheter body includes a sidewall having at least one opening therethrough, and the at least one wire anchor may be deployed through the at least one opening. Alternatively, the catheter body may include an opening at a distal end thereof, and the at least one wire anchor may be deployable through the opening at the distal end of the catheter body. Of course, the catheter body may also include at least one lumen through which the wire anchor is introduced to be deployed through the at least one opening.

The at least one wire anchor may include at least one wire loop. Alternatively, the at least one wire anchor may include at least one pigtail wire anchor. In still other examples of the teachings, the at least one wire anchor may include at least one wire anchor helically wound about the catheter body. The at least one wire anchor may also include at least one wire basket, which may be deployed from the distal end of the catheter.

According to another aspect of the teachings, a catheter for anchoring an electrode in a coronary sinus includes: an elongate catheter body having a central axis and a flexible anchor segment, the flexible anchor segment being movable between a deployed configuration, wherein the flexible anchor segment is deviated from the central axis of the catheter body to engage a tissue surface of the coronary sinus such that relative movement between the catheter body and the coronary sinus is inhibited without completely occluding the coronary sinus, and an undeployed configuration, wherein the flexible anchor segment is generally collinear with the central axis of the catheter body to introduce the catheter into the coronary sinus; and at least one electrode on the catheter body.

In some examples of the teachings, the flexible anchor segment is biased into the undeployed configuration, and the catheter further includes a tension member. By placing the tension member in tension, the flexible anchor segment may be moved into the deployed configuration. In other examples of the teachings, the flexible anchor segment is biased into the deployed configuration, and a sheath, stylet, guide wire, or other suitable straightening device may be used to move the flexible anchor segment into the undeployed configuration. Of course, the flexible anchor segment may be positioned as desired along the catheter body, including within an intermediate section of the catheter body or at the distal end of the catheter body.

The present teachings also provides a method of generating a cardiac geometry, including the steps of: providing a coronary sinus catheter having an anchor structure and an electrode thereon; introducing the coronary sinus catheter into the coronary sinus; deploying the anchor structure to engage a tissue surface of the coronary sinus, thereby inhibiting relative movement between the coronary sinus catheter and the coronary sinus; and conducting a cardiac mapping operation using the electrode on the coronary sinus catheter as a reference electrode.

An advantage of the present invention is that it permits a reference electrode to be positively anchored within a vessel, thereby facilitating creation of anatomical geometries.

Another advantage of the present invention is that it positively anchors a reference electrode within a vessel while preserving perfusion through the vessel, thereby minimizing stasis and thrombus creation and enhancing dwell time.

The foregoing and other aspects, features, details, utilities, and advantages of the present invention will be apparent from reading the following description and claims, and from reviewing the accompanying drawings.

### BRIEF DESCRIPTION OF THE DRAWINGS

The Figures do not show all features as combined by claim 1, respectively.
Fig. 1 is a schematic diagram of a localization system utilized in an electrophysiology study.
Fig. 2 depicts an exemplary mapping catheter utilized in an electrophysiology study.
Fig. 3 illustrates a catheter carrying a fixed reference electrode positioned in the coronary sinus.
Fig. 4 illustrates an embodiment of a catheter to anchor an electrode in a vessel, such as the coronary sinus, having expandable segments to provide bias against the vessel wall.
Fig. 5 illustrates another embodiment of a catheter to anchor an electrode in a vessel, such as the coronary sinus, including a balloon to provide bias against the vessel wall.
Fig. 6 is a close-up view of the anchor section of another embodiment of a catheter to anchor an electrode in a vessel, such as the coronary sinus, having a wire basket to provide bias against the vessel wall.
Fig. 7 illustrates an example of a wire anchor catheter to anchor an electrode in a vessel, such as the coronary sinus, having wire loops to provide bias against the vessel wall.
Fig. 8 is a close-up view of the wire loops of the catheter illustrated in Fig. 7.
Fig. 9 depicts the proximal end of a catheter according to some examples of the present teachings.
Fig. 10 is still another example of a wire anchor catheter to anchor an electrode in a vessel, such as the coronary sinus, having a pigtail anchor to provide bias against the vessel wall.
Fig. 11 depicts another example of a wire anchor catheter to anchor an electrode in a vessel, such as the coronary sinus, illustrating use of a helically wound wire to provide bias against the vessel wall.
Fig. 12 shows a further example of a wire anchor catheter to anchor an electrode in a vessel, such as the coronary sinus, where a conical, helically-wound wire may be deployed from the distal end of the catheter to provide bias against the vessel wall.
Fig. 13 illustrates another example of a wire anchor catheter to anchor an electrode in a vessel, such as the coronary sinus, where a wire basket may be deployed from the distal end of the catheter to provide bias against the vessel wall.
Fig. 14 illustrates an alternative example of the wire anchor catheter shown in Fig. 13.
Fig. 15 depicts a catheter to anchor an electrode in a vessel, such as the coronary sinus, illustrating use of a balloon-tipped wire deployed from the distal end of the catheter to provide bias against the vessel wall.
Fig. 16 depicts another example of a catheter to anchor an electrode in a vessel, such as the coronary sinus, illustrating a spiral-wound wire deployed from the distal end of the catheter to provide bias against the vessel wall.
Figs. 17a and 17b depict, in plan and perspective view, respectively, a catheter according to another aspect of the teachings invention having a flexible anchor section that provides bias against a vessel wall.
Fig. 18 illustrates the bias against the vessel wall provided by the catheter of Figs. 17a and 17b.
Figs. 19 and 20 illustrate alternative examples embodiments of a catheter having a flexible anchor section that provides bias against a vessel wall.
Figs. 21 through 24 illustrate several catheter and sheath assemblies according to the present teachings that may be used to introduce an electrode into a vessel, such as the coronary sinus, and then anchor the electrode within the vessel.
Figs. 25 and 26 illustrate a catheter and stylet assembly according to another aspect of the present teachings that may be used to introduce an electrode into a vessel, such as the coronary sinus, and then anchor the electrode within the vessel, in the undeployed and deployed configurations, respectively.
Figs. 27 and 28 are free-body diagrams of actuation forces that may be used to actuate a catheter according to some examples of the present teachings between the undeployed configuration and the deployed configuration.
Figs. 29 through 31 depict several actuation mechanisms to actuate a flexible anchor section of a catheter according to some aspects of the present teachings.
Fig. 32 depicts the catheter of Fig. 5 deployed within a vessel and illustrates perfusion across the balloon.
Figs. 33 and 34 depict a catheter to anchor an electrode in a vessel, such as the coronary sinus, according to still another example of the present teachings, where bias is provided by one or more expandable mesh sections.
Figs. 35 and 36 illustrate an alternative catheter and stylet assembly according to another aspect of the present teachings that may be used to introduce an electrode into a vessel, such as the coronary sinus, and then anchor the electrode within the vessel, in the undeployed and deployed configurations, respectively.
Fig. 37 depicts a catheter to anchor an electrode in a vessel according to yet another example of the present teachings, including roughened surfaces to provide friction between the catheter and the vessel wall.
Figs 38 through 40 illustrate a balloon catheter that may be anchored within a vessel, such as the coronary sinus, to substantially completely occlude the vessel.
Figs. 41 and 42 illustrate, in side and end view, respectively, a catheter to anchor an electrode in a vessel, such as the coronary sinus, including a plurality of balloons.

### DETAILED DESCRIPTION

The present invention provides apparatus for positioning and retaining (that is, anchoring) a reference electrode for use in a localization system. Such systems are often employed in procedures carried out within a human body, and in particular in cardiac diagnostic and therapeutic procedures. Therefore, for purposes of illustration, the invention will be described in detail in the context of a localization system utilized in a cardiac electrophysiology procedure. It is contemplated, however, that the present invention may be practiced to good advantage in other contexts.

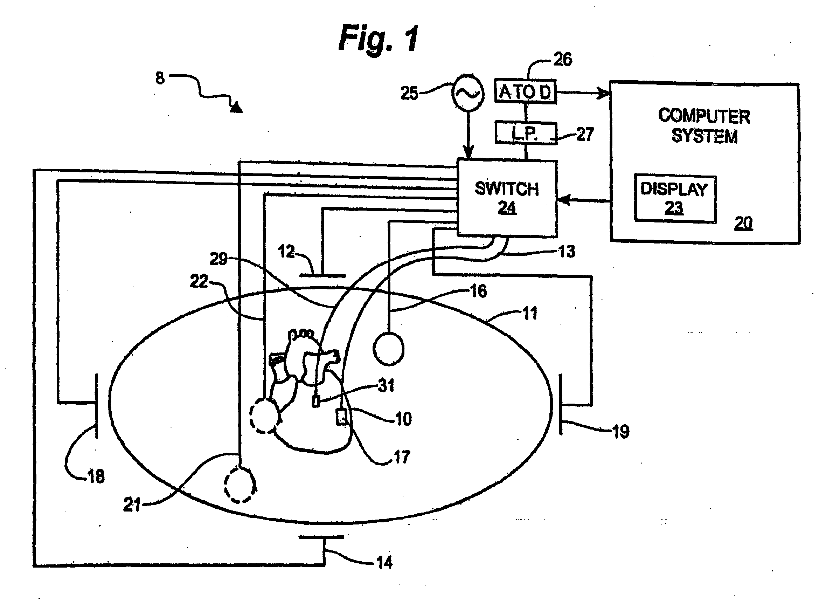

Fig. 1 shows a schematic diagram of a localization system 8 for conducting cardiac electrophysiology studies by navigating a cardiac catheter and measuring electrical activity occurring in a heart 10 of a patient 11 and three-dimensionally mapping the electrical activity and/or information related to or representative of the electrical activity so measured. System 8 can be used, for example, to create an anatomical model of the patient's heart 10 using one or more electrodes. System 8 can also be used to measure electrophysiology data at a plurality of points along a cardiac surface, and store the measured data in association with location information for each measurement point at which the electrophysiology data was measured, for example to create a diagnostic data map of the patient's heart 10. As one of ordinary skill in the art will recognize, and as will be further described below, localization system 8 determines the location of objects, typically within a three-dimensional space, and expresses those locations as position information determined relative to at least one reference.

For simplicity of illustration, the patient 11 is depicted schematically as an oval. Three sets of surface electrodes (e.g., patch electrodes) are shown applied to a surface of the patient 11, defining three generally orthogonal axes, referred to herein as an x-axis, a y-axis, and a z-axis. The x-axis surface electrodes 12, 14 are applied to the patient along a first axis, such as on the lateral sides of the thorax region of the patient (e.g., applied to the patient's skin underneath each arm) and may be referred to as the Left and Right electrodes. The y-axis electrodes 18, 19 are applied to the patient along a second axis generally orthogonal to the x-axis, such as along the inner thigh and neck regions of the patient, and may be referred to as the Left Leg and Neck electrodes. The z-axis electrodes 16, 22 are applied along a third axis generally orthogonal to both the x-axis and the y-axis, such as along the sternum and spine of the patient in the thorax region, and may be referred to as the Chest and Back electrodes. The heart 10 lies between these pairs of surface electrodes 12/14, 18/19, and 16/22.

An additional surface reference electrode (e.g., a "belly patch") 21 provides a reference and/or ground electrode for the system 8. The belly patch electrode 21 may be an alternative to a fixed intra cardiac electrode 31, described in further detail below. It should also be appreciated that, in addition, the patient 11 may have most or all of the conventional electrocardiogram (ECG) system leads in place. This ECG information is available to the system 8, although not illustrated in Fig. 1.

A representative catheter 13 having at least one electrode 17 (e.g., a distal electrode) is also shown. This representative catheter electrode 17 is referred to as the "roving electrode," "moving electrode," or "measurement electrode" throughout the specification. Typically, multiple electrodes on catheter 13, or on multiple such catheters, will be used. In one example, for example, localization system 8 may comprise up to sixty-four electrodes on up to twelve catheters disposed within the heart and/or vasculature of the patient. Of course, this example is merely exemplary, and any number of electrodes and catheters may be used.

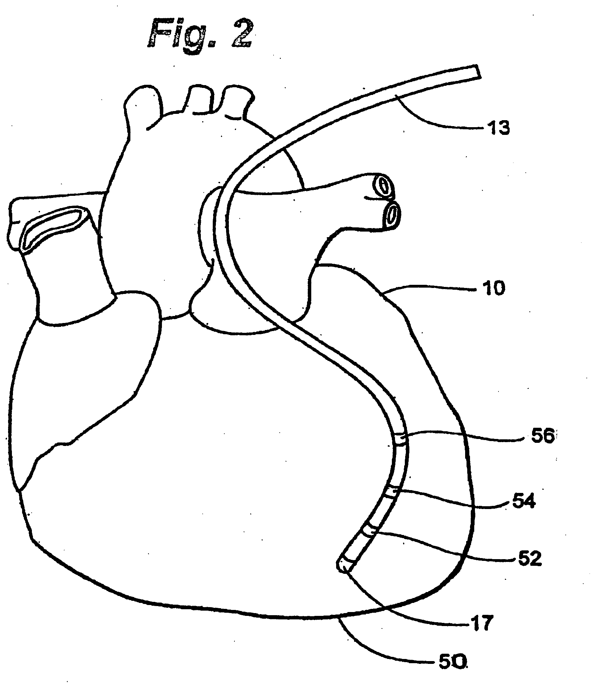

For purposes of this disclosure, an exemplary catheter 13 is shown in Fig. 2. In Fig. 2, catheter 13 extends into the left ventricle 50 of the patient's heart 10. Catheter 13 includes electrode 17 on its distal tip, as well as a plurality of additional measurement electrodes 52, 54, 56 spaced along its length. Typically, the spacing between adjacent electrodes will be known, though it should be understood that the electrodes may not be evenly spaced along catheter 13 or of equal size to each other. Since each of these electrodes 17, 52, 54, 56 lies within the patient, location data may be collected simultaneously for each of the electrodes by localization system 8.

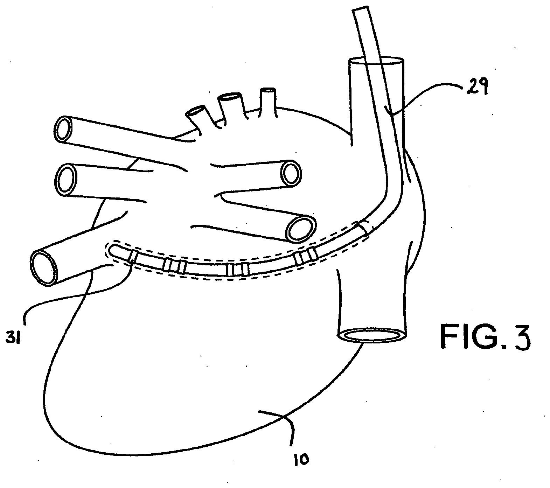

Returning now to Fig. 1, an optional fixed reference electrode 31 (e.g., attached to a wall of the heart 10) is shown on a second catheter 29. For calibration purposes, this electrode 31 may be stationary (e.g., attached to or near the wall of the heart) or disposed in a fixed spatial relationship with the roving electrodes (e.g., electrodes 17, 52, 54, 56), and thus may be referred to as a "navigational reference" or "local reference." The fixed reference electrode 31 may be used in addition or alternatively to the surface reference electrode 21 described above. In many instances, a coronary sinus electrode or other fixed electrode in the heart 10 can be used as a reference for measuring voltages and displacements; that is, as described below, fixed reference electrode 31 may define the origin of a coordinate system. This is illustrated, for example, in Fig. 3, which depicts second catheter 29, including multiple fixed reference electrodes 31, anchored within the coronary sinus.

Each surface electrode is coupled to the multiplex switch 24, and the pairs of surface electrodes are selected by software running on a computer 20, which couples the surface electrodes to a signal generator 25. The computer 20, for example, may comprise a conventional general-purpose computer, a special-purpose computer, a distributed computer, or any other type of computer. The computer 20 may comprise one or more processors, such as a single central processing unit (CPU), or a plurality of processing units, commonly referred to as a parallel processing environment, which may execute instructions to practice the various aspects of the present teachings described herein.

Generally, three nominally orthogonal electric fields are generated by a series of driven and sensed electric dipoles (e.g., surface electrode pairs 12/14, 18/19, and 16/22) in order to realize catheter navigation in a biological conductor. Alternatively, these orthogonal fields can be decomposed and any pairs of surface electrodes can be driven as dipoles to provide effective electrode triangulation. Additionally, such non-orthogonal methodologies add to the flexibility of the system. For any desired axis, the potentials measured across the roving electrodes resulting from a predetermined set of drive (source-sink) configurations may be combined algebraically to yield the same effective potential as would be obtained by simply driving a uniform current along the orthogonal axes.

Thus, any two of the surface electrodes 12, 14, 16, 18, 19, 22 may be selected as a dipole source and drain with respect to a ground reference, such as belly patch 21, while the unexcited electrodes measure voltage with respect to the ground reference. The roving electrodes 17, 52, 54, 56 placed in the heart 10 are exposed to the field from a current pulse and are measured with respect to ground, such as belly patch 21. In practice the catheters within the heart may contain more or fewer electrodes than the four shown, and each electrode potential may be measured. As previously noted, at least one electrode may be fixed to the interior surface of the heart to form a fixed reference electrode 31, which is also measured with respect to ground, such as belly patch 21, and which may be defined as the origin of the coordinate system relative to which localization system 8 measures positions. Data sets from each of the surface electrodes, the internal electrodes, and the virtual electrodes may all be used to determine the location of the roving electrodes 17, 52, 54, 56 within heart 10.

The measured voltages may be used to determine the location in three-dimensional space of the electrodes inside the heart, such as roving electrodes 17, 52, 54, 56, relative to a reference location, such as reference electrode 31. That is, the voltages measured at reference electrode 31 may be used to define the origin of a coordinate system, while the voltages measured at roving electrodes 17, 52, 54, 56 may be used to express the location of roving electrodes 17, 52, 54, 56 relative to the origin. Preferably, the coordinate system is a three-dimensional (x, y, z) Cartesian coordinate system, though the use of other coordinate systems, such as polar, spherical, and cylindrical coordinate systems, is within the teachings.

As should be clear from the foregoing discussion, the data used to determine the location of the electrode(s) within the heart is measured while the surface electrode pairs impress an electric field on the heart. The electrode data may also be used to create a respiration compensation value used to improve the raw location data for the electrode locations as described in U.S. Patent Application Publication No. 2004/0254437. The electrode data may also be used to compensate for changes in the impedance of the body of the patient as described in co-pending U.S. Patent Application No. 11/227,580, filed on 15 September 2005.

In summary, the system 8 first selects a set of surface electrodes and then drives them with current pulses. While the current pulses are being delivered, electrical activity, such as the voltages measured at least one of the remaining surface electrodes and in vivo electrodes, is measured and stored. Compensation for artifacts, such as respiration and/or impedance shifting, may be performed as indicated above.

In a preferred example, the localization/mapping system is the EnSite NavX™ navigation and visualization system of St. Jude Medical, Atrial Fibrillation Division, Inc., which generates the electrical fields described above. Other localization systems, however, may be used in connection with the present teachings, including for example, the CARTO navigation and location system of Biosense Webster, Inc., or the AURORA® system of Northern Digital Inc., both of which utilize magnetic fields rather than electrical fields. The localization and mapping systems described in the following patents can also be used with the present teachings: United States Patent Nos. 6,990,370; 6,978,168; 6,947,785; 6,939,309; 6,728,562; 6,640,119; 5,983,126; and 5,697,377.

The fields generated by localization system 8, whether an electrical field (e.g., EnSite NavX™), a magnetic field (e.g., CARTO, AURORA®), or another suitable field, may be referred to generically as "localization fields," while the elements generating the fields, such as surface electrodes 12, 14, 16, 18, 19, and 22 may be generically referred to as "localization field generators." As described above, surface electrodes 12, 14, 16, 18, 19, and 22 may also function as detectors to measure the characteristics of the localization field (e.g., the voltages measured at roving electrodes 17, 52, 54, 56). Though the present teachings will be described primarily in the context of a localization system that generates an electrical field, one of ordinary skill in the art will understand how to apply the principles disclosed herein in other types of localization fields (e.g., by replacing electrodes 17, 52, 54, 56 with coils to detect different components of a magnetic field).

As described above, localization system 8 may employ one or more reference electrodes 31, carried on one or more catheters 29, as a reference for the three-dimensional coordinate system of localization system 8. Accordingly, it is desirable for reference electrodes 31 to be positively retained (often referred to as "anchored") at the desired location for the reference of the three-dimensional coordinate system, often within the coronary sinus.

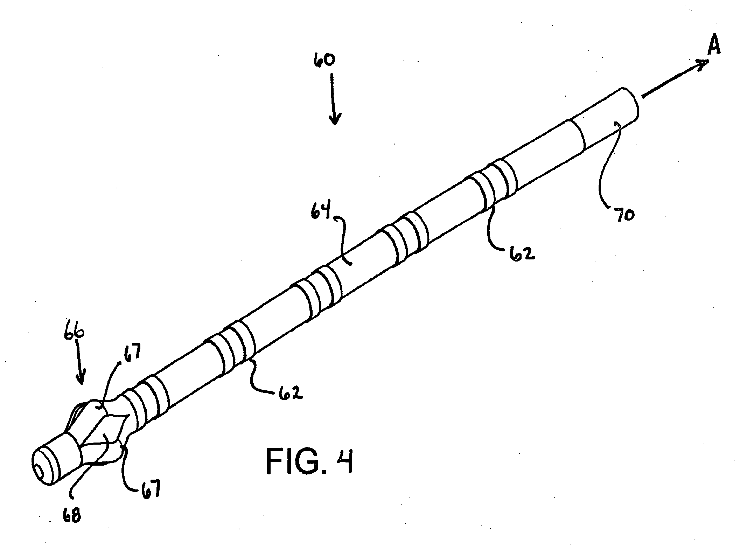

Fig. 4 depicts an embodiment of a catheter 60 for positioning and retaining (that is, anchoring) one or more electrodes 62 within a coronary sinus, for example for use as reference electrode 31. Catheter 60 generally includes an elongate catheter body 64 adapted (that is, sized and dimensioned) to be inserted into a coronary sinus. At least one electrode 62, such as at least one ring electrode, is provided on catheter body 64.

Catheter body 64 also includes an anchor section 66 having an expandable axial cross-section. Anchor section 66 may be actuated between an undeployed configuration, wherein the expandable axial cross-section of anchor section 66 is in a collapsed state generally co-extensive with at least the portion of catheter body 64 adjacent anchor section 66, and a deployed configuration, wherein the expandable axial cross-section of anchor section 66 is in an expanded state larger than at least the portion of catheter body 64 adjacent anchor section 66. In general, the undeployed configuration is utilized to introduce catheter 60 into and remove catheter 60 from the patient, while the deployed configuration is utilized to stabilize catheter 60 within a vessel during an electrophysiology procedure. Electrodes 62 may be positioned proximally of anchor section 66, distally of anchor section 66, and/or on anchor section 66

In the embodiment illustrated in Fig. 4, the expandable axial cross-section of anchor section 66 is provided by a plurality of expandable segments 67 arranged around the circumference of catheter body 64. When anchor section 66 is in the undeployed configuration (not illustrated), expandable segments 67 are retracted to be generally co-extensive with catheter body 64, such that the axial cross-section of anchor section 66 is suitable to introduce catheter 60 into the coronary sinus. When anchor section 66 is in the deployed configuration (illustrated in Fig. 4), expandable segments 67 flex outwardly from catheter body 64 so as to engage a tissue surface of the coronary sinus (e.g., the inner wall of the vessel).

Contact between anchor section 66 and the tissue surface of the coronary sinus creates opposing frictional forces, also known as "bias," that advantageously inhibit movement between catheter body 64 and the coronary sinus. By inhibiting movement between catheter body 64 and the coronary sinus, one or more of electrodes 62 may be used as a fixed reference electrode 31 for localization system 8 as described above.

Catheter 60 may also include an actuation mechanism, such as tension member 70 or another suitable mechanism, operably coupled to anchor section 66 to actuate anchor section 66 between the undeployed configuration and the deployed configuration. For example, placing tension member 70 in tension by pulling in the direction of arrow A may cause anchor section 66 to assume the deployed configuration, while releasing tension may cause anchor section 66 to return to the undeployed configuration. Of course, the proximal end of tension member 70 may be connected to a handle or the like to facilitate placing tension member 70 in tension.

It is also desirable to maintain one or more perfusion pathways, such as gaps 68 between adjacent segments 67, when anchor section 66 is in the deployed configuration. These perfusion pathways permit blood flow through the coronary sinus from one side of anchor section 66 to the other around the exterior of catheter 60. This prevents catheter 60 from completely occluding the coronary sinus, even with anchor segment 66 in the deployed configuration, thereby minimizing stasis and thrombus creation and advantageously increasing dwell time of catheter 60 within the coronary sinus during a cardiac mapping operation.

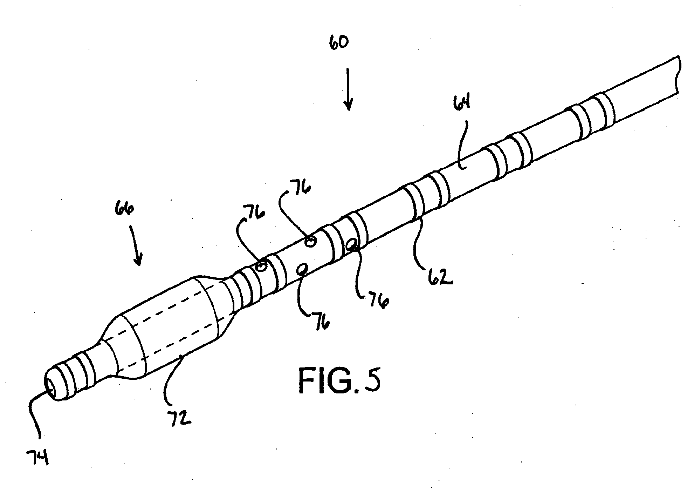

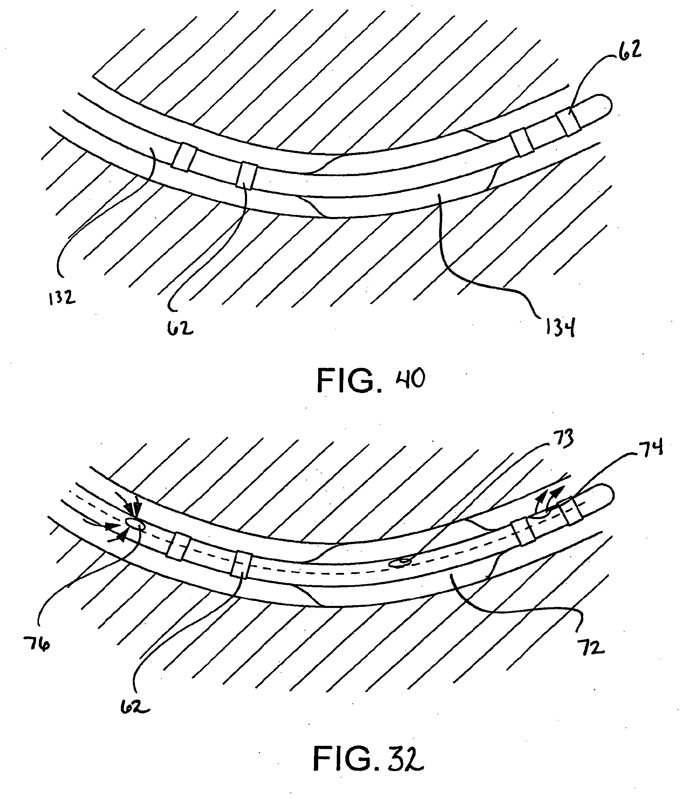

Fig. 5 illustrates a second embodiment of a catheter 60 having an anchor section 66 for anchoring one or more electrodes 62 within a coronary sinus. In the embodiment illustrated in Fig. 5, anchor section 66 includes at least one balloon 72 positioned about a circumference of catheter body 64. Balloon 72 is fluidly coupled to an inflation fluid source (not shown), in order to inflate balloon 72 from the undeployed configuration (not illustrated) into the deployed configuration (illustrated in Fig. 5), for example through inflation port 73 (shown in Fig. 32). Perfusion pathways may be provided by one or more perfusion passages through the interior of catheter 60, each of which includes a first opening 74 positioned distally of anchor section 66 (e.g., distally of balloon 72) and a second opening 76 positioned proximally of anchor section 66 (e.g., proximally of balloon 72). In addition, perfusion pathways are provided by altering the shape of balloon 72 such that it does not completely occlude the coronary sinus, thereby permitting perfusion around the exterior of catheter 60.

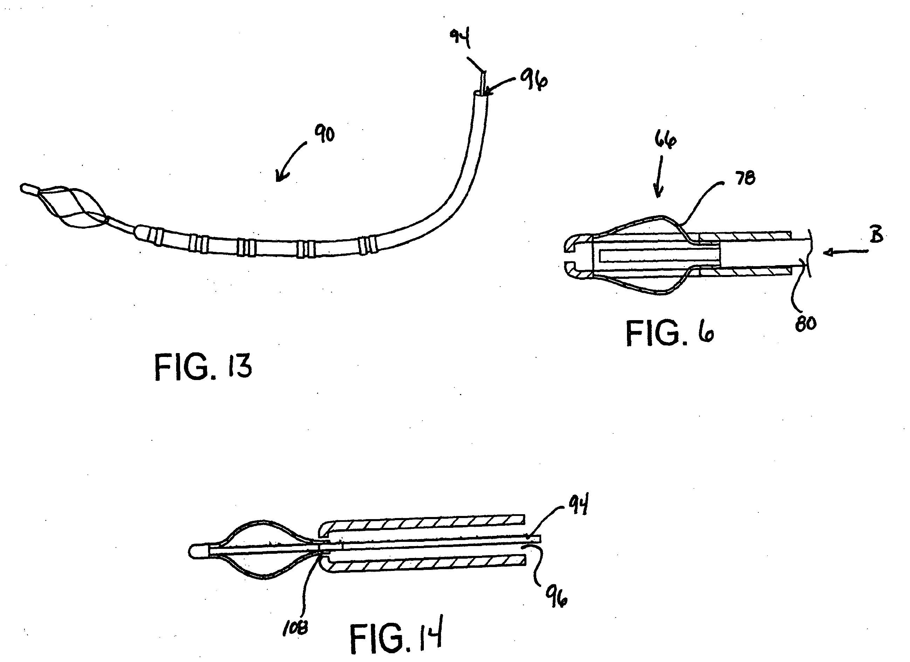

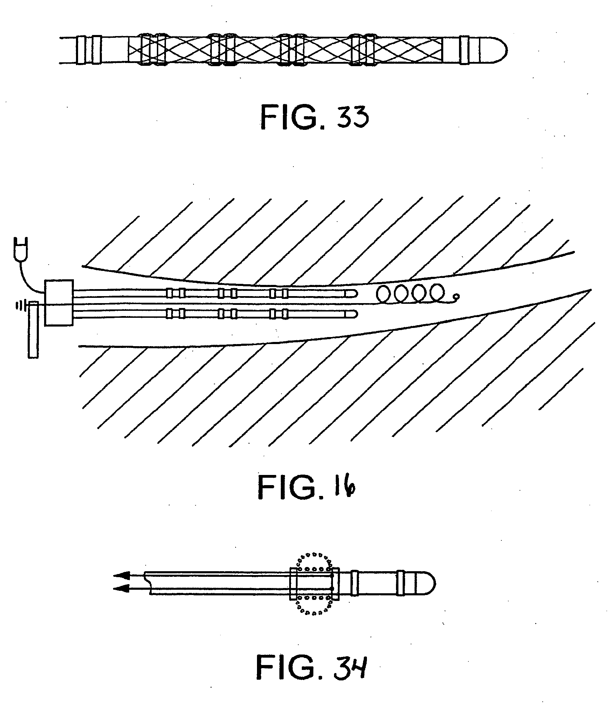

It is contemplated that other suitable expandable members, such as a wire basket 78 as illustrated in Fig. 6, may be used in place of balloon 72. Wire basket 78 may be connected to a basket actuator 80, which, when moved in the direction of arrow B, deploys wire basket 78 into the deployed configuration, and when moved opposite the direction of arrow B, returns wire basket 78 to the undeployed configuration. One of ordinary skill in the art will appreciate that wire basket 78 in the deployed configuration may resemble expandable segments 67 in the deployed configuration, such that perfusion pathways are provided around the exterior of catheter 60. According to claim 1, perfusion passages through the interior of catheter 60 are provided in addition to perfusion pathways around the exterior of catheter 60. Other suitable expandable members include expandable coiled mesh sleeves, such as illustrated in Figs. 33 and 34.

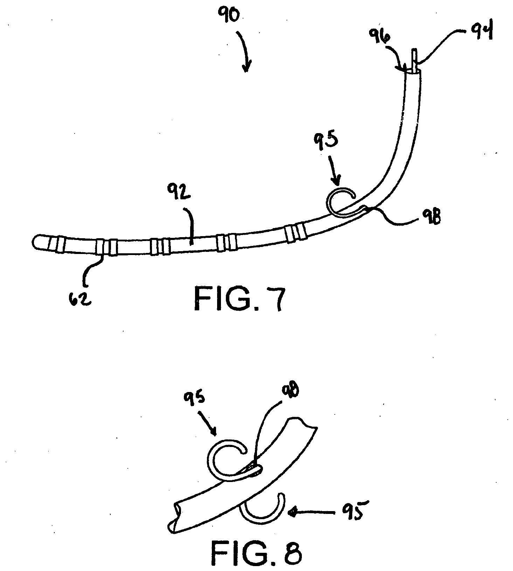

Fig. 7 depicts a further aspect of the present teachings. As shown in Fig. 7, a catheter 90 for anchoring an electrode in a coronary sinus generally includes an elongate catheter body 92 adapted to be inserted into a coronary sinus, at least one wire anchor 94 coupled to catheter body 92, and at least one electrode 62 on the catheter body.

Wire anchor 94 is movable between an undeployed configuration and a deployed configuration. With wire anchor 94 in the undeployed configuration, catheter 90 is movable relative to the coronary sinus, for example in order to be introduced into and/or removed from the coronary sinus. When in the deployed configuration, wire anchor 94 engages a tissue surface of the coronary sinus (e.g., the inner wall of the vessel) and creates bias that inhibits movement between the catheter body and the coronary sinus. Because it does not fully occlude the coronary sinus, wire anchor 94 advantageously preserves perfusion through the coronary sinus.

In some examples of the teachings, as illustrated in Figs. 7 and 8, wire anchor 94 terminates in a wire loop 95, which may be pre-formed by using a shape memory alloy such as nickel-titanium (e.g., Nitinol) as wire anchor 94. One or more such wire loops 95 may be used to capture the tissue of the coronary sinus, preferably at or near the ostium of the coronary sinus. Of course, additional wire loops 95 may be employed to increase the probability of tissue capture.

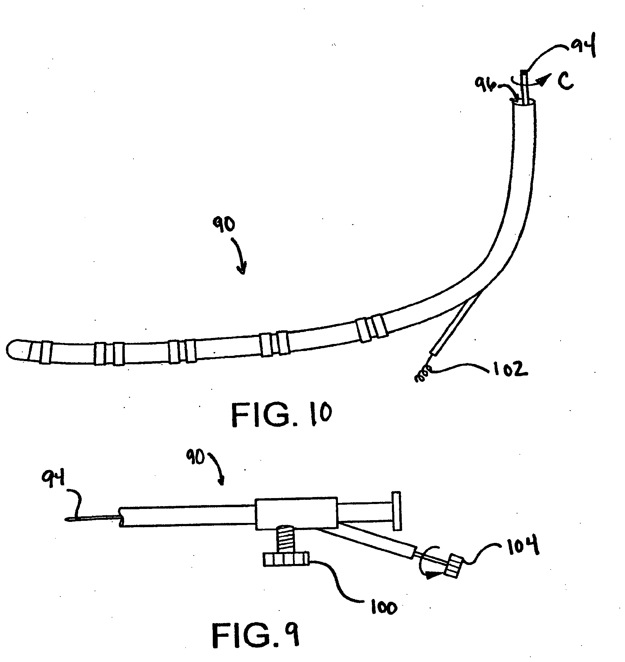

As shown in Fig. 7, catheter 90 may include a lumen 96 therethrough and at least one opening 98 through a sidewall of catheter body 92. Wire anchor 94 may be introduced through lumen 96 and deployed through opening 98. One of ordinary skill in the art will recognize that, even with wire anchor 94 in the deployed configuration, a small amount of movement may be possible between catheter body 92 and wire anchor 94. This "play" may be advantageously used to "fine tune" the position of catheter 90 and electrodes 62 within the coronary sinus. Once catheter 90 and electrodes 62 are positioned as desired, one or more wire locks 100 (Fig. 9) may be employed to couple the proximal ends of wire anchors 94 to the proximal end of catheter 90 and positively restrain wire anchor 94 relative to catheter body 92.

Fig. 10 depicts an alternative example of catheter 90. In the example illustrated in Fig. 10, wire anchor 94 is introduced through lumen 96 and deployed through opening 98, and terminates in a pigtail anchor 102. It is contemplated that pigtail anchor 102 may be deployed by rotating wire anchor 94 (arrow C in Fig. 10), for example by using a pigtail drive 104 (shown in Fig. 9).

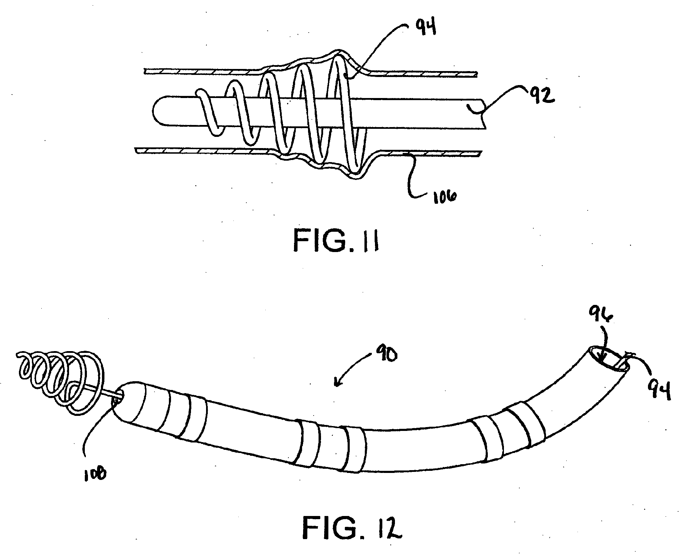

Of course, wire anchor 94 may also be mounted to and deployed from the outside of catheter body 92, for example by entrapping one or more wire anchors in a sheath to introduce the catheter and then removing the sheath to deploy the wire anchors. Alternatively, wire anchor 94 may be helically wound about catheter body 92, as illustrated in Fig. 11. When unwound into the deployed configuration, wire anchor 94 engages vessel wall 106 to create bias. Wire anchor 94 may be returned to the undeployed configuration by winding it back about catheter body 92.

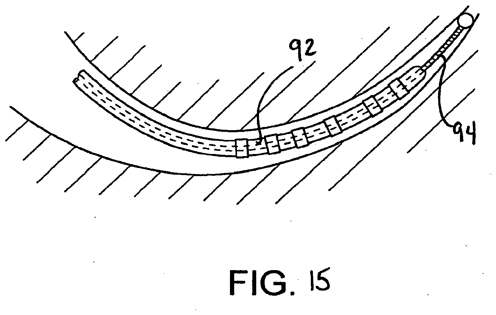

In still other examples, wire anchor 94 may be introduced through lumen 96 and deployed from a distal tip opening 108. Of course, wire anchor may terminate in any desired configuration, such as a conical helix (Fig. 12); a wire basket (Fig. 13), which may be self-expanding; a flat-wire basket (Fig. 14), which may be self-expanding; a wire-mounted balloon (Fig. 15); spiral coils (Fig. 16); or any other suitable configuration.

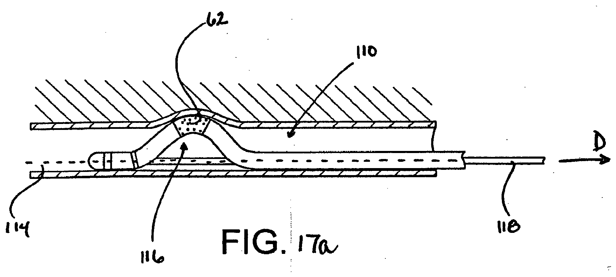

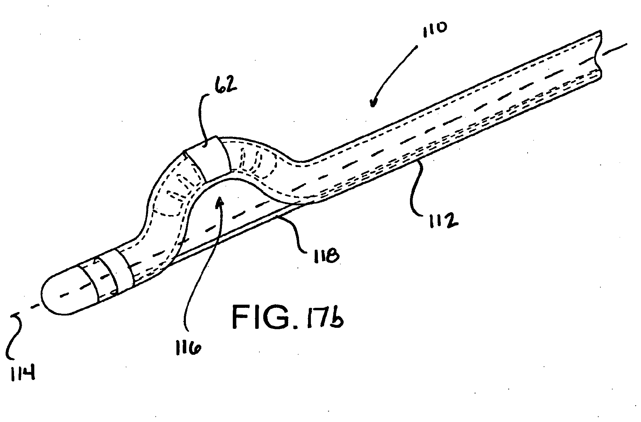

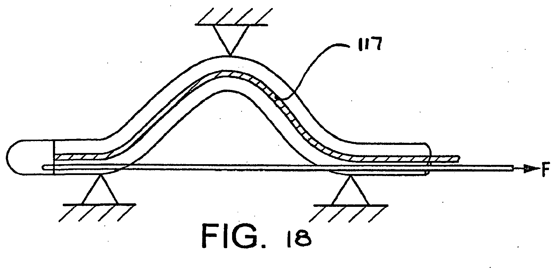

Yet another aspect of the present teachings is illustrated in Figs. 17a (plan view) and 17b (perspective view). As shown in Figs. 17a and 17b, a catheter 110 for anchoring an electrode (e.g., electrode 62) in a coronary sinus generally includes an elongate catheter body 112 having a central axis 114 (shown in dashed line) and a flexible anchor segment 116. Flexible anchor segment 116 is movable between an undeployed configuration (not shown) and a deployed configuration (shown in Figs. 17a and 17b). In the undeployed configuration, flexible anchor segment 116 is generally collinear with central axis 114 of catheter body 112 such that catheter 110 may be introduced into the coronary sinus. In the deployed configuration, flexible anchor segment 116 is deviated from central axis 114 so as to engage the tissue surface of the coronary sinus (e.g., the inner wall of the vessel), thereby creating bias (shown in Fig. 18) and inhibiting relative movement between catheter 110 (and therefore electrode 62) and the coronary sinus.

In some examples of the teachings, flexible anchor segment 116 is predisposed or biased into the undeployed configuration, for example through the use of a shaping member such as a shape memory wire "backbone" 117 (Fig. 18). Thus, an actuation member, such as tension member 118, may be provided in order to actuate flexible anchor segment 116 into the deployed configuration. As shown in Fig. 17a, pulling on tension member 118 in the direction of arrow D puts tension member 118 in tension and actuates flexible anchor segment 116 into the deployed configuration; releasing tension causes flexible anchor segment 116 to return to the undeployed configuration.

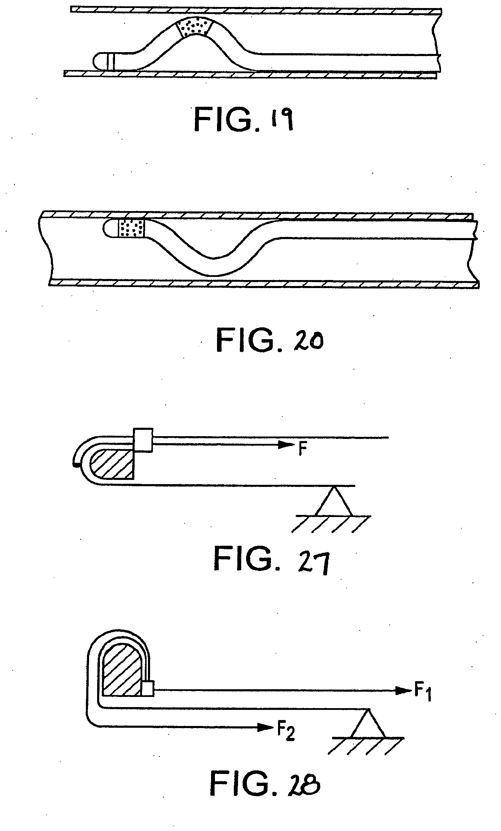

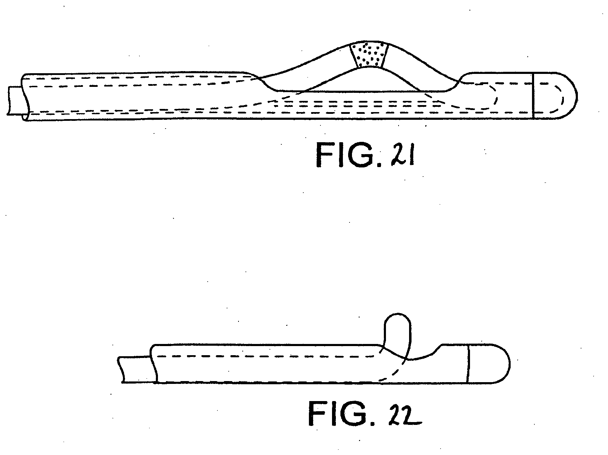

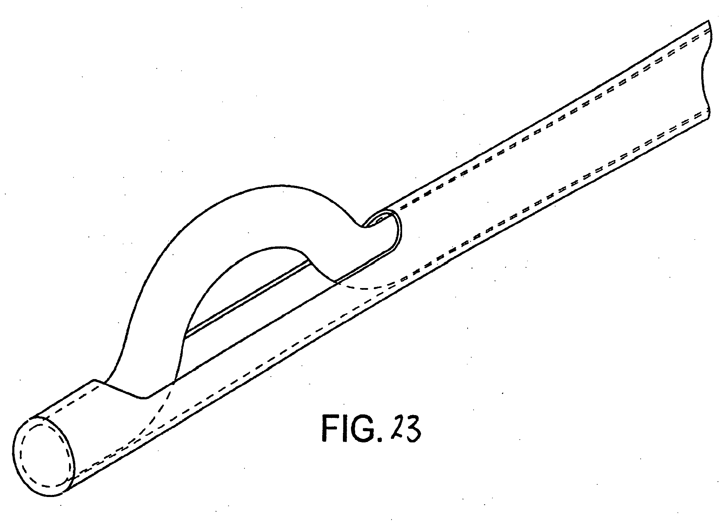

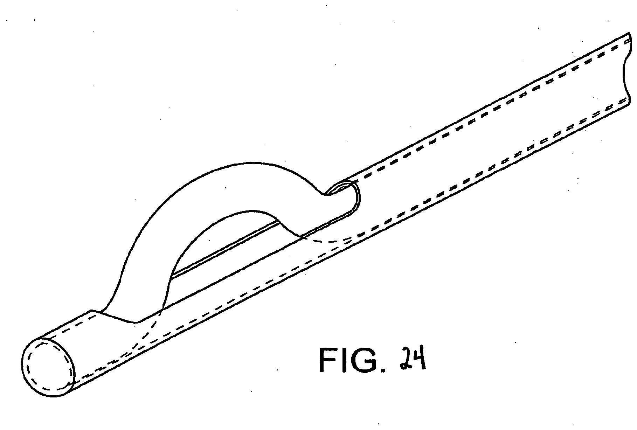

In still other examples, flexible anchor segment 116 may be predisposed or biased into the deployed configuration, for example as shown in Figs. 19 and 20. Flexible anchor segment 116 may be straightened into the undeployed configuration for introduction into the coronary sinus through the use of a sheath, for example as shown in Figs. 21-24.

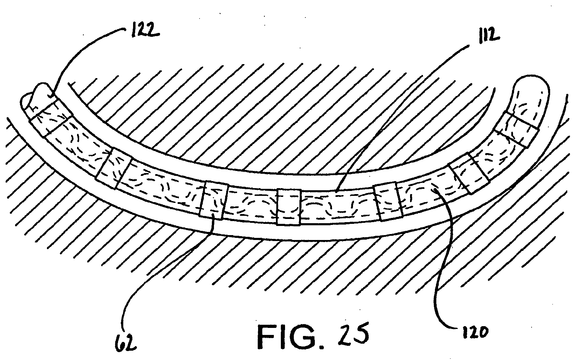

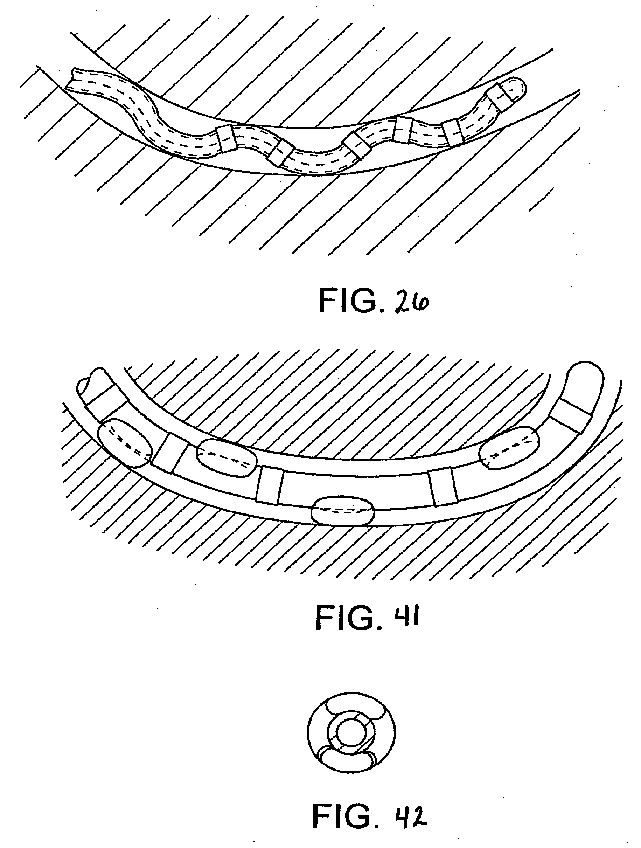

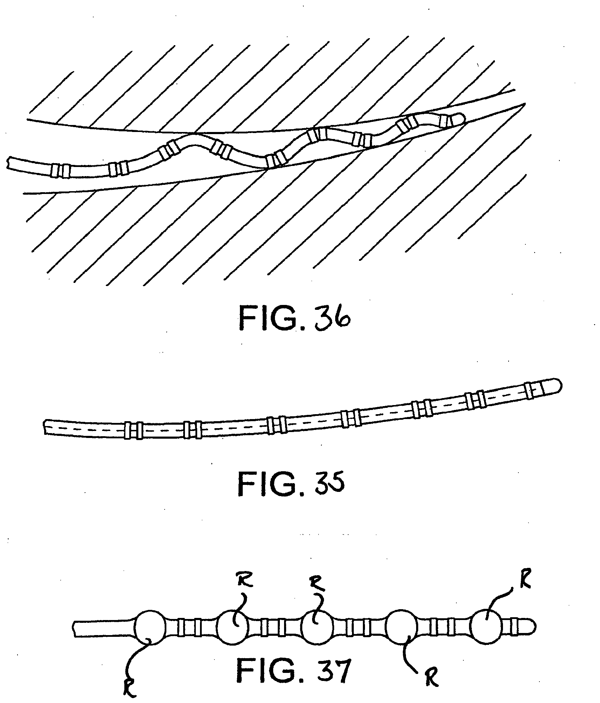

Alternatively, catheter body 112 may include a plurality of flexible anchor segments 116, for example as illustrated in Figs. 25 and 26. As shown in Fig. 25, flexible anchor segments 116 are predisposed or biased into the deployed configuration by a shaping member 120 disposed within catheter body 112. A stylet 122 may be inserted into catheter body 112 in order to attain the undeployed configuration. When stylet 122 is removed, flexible anchor segments 116 return to the deployed configuration and engage the tissue surface of the coronary sinus (Fig. 26), thereby creating bias and inhibiting movement between catheter 110 and the coronary sinus. This is also illustrated in Fig. 35 (undeployed configuration using a stylet, guidewire, or other suitable straightening device), and Fig. 36 (deployed configuration showing multiple flexible anchor segments, which may be in plane, corkscrew-shaped, or another suitable configuration.

It should be understood that flexible anchor segment or segments 116 may be positioned as desired along catheter body 112. For example, in some examples of the teachings, at least one flexible anchor segment is positioned within an intermediate section of catheter body 112, as illustrated in Figs. 19 and 20. In still other examples of the teachings, a flexible anchor segment is positioned at a distal end of catheter body 112, as illustrated in Fig. 22.

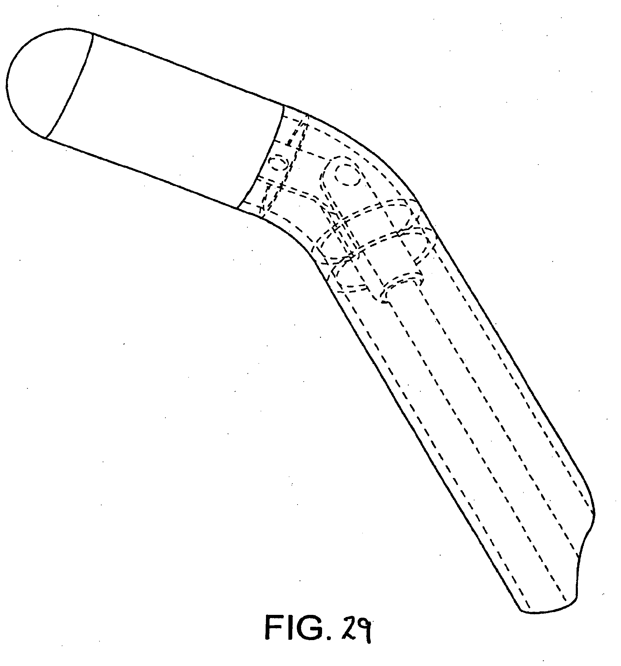

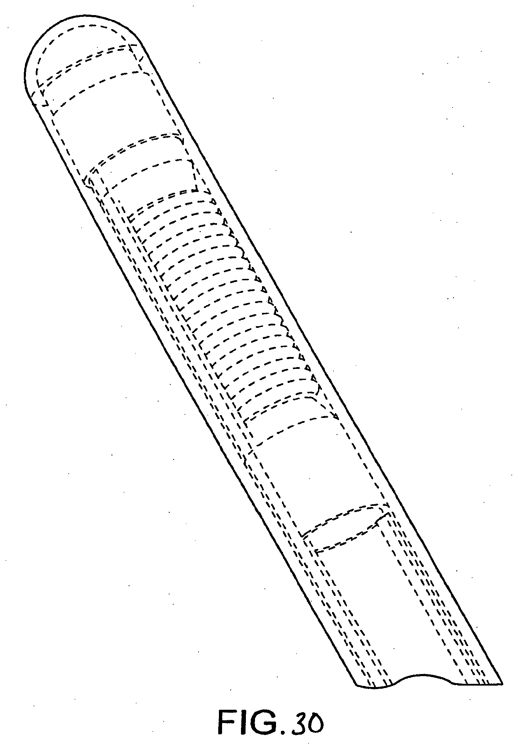

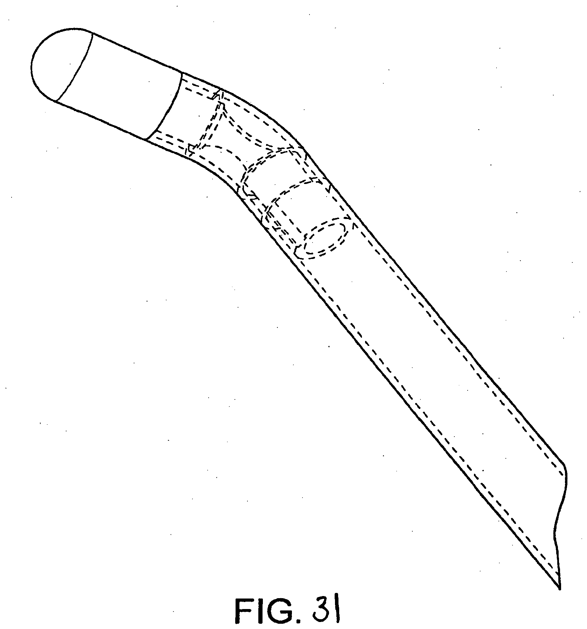

It should also be understood that there are a number of suitable ways to actuate flexible anchor segment 116 between the undeployed configuration and the deployed configuration. For example, Figs. 27 and 28 illustrate two free-body diagrams of forces that may be applied to flexible anchor segment 116, for example through tension members, in order to actuate it between the undeployed configuration and the deployed configuration. Figs. 29-31 illustrate various actuating mechanisms for a flexible anchor segment positioned at the distal end of catheter body 112.

The devices and methods disclosed herein may be practiced to good advantage in generating a cardiac geometry. A coronary sinus catheter having an anchor structure and an electrode may be provided and introduced into the coronary sinus. Once the catheter is positioned as desired, the anchor structure may be deployed to engage a tissue surface of the coronary sinus, thereby inhibiting relative movement between the coronary sinus catheter (and therefore any electrodes thereon) and the coronary sinus. The anchor structure may be any of the structures disclosed herein (e.g., sections of the catheter body having an expandable axial cross-section, wire anchors, anchor segments of the catheter body, and the like). With the electrodes so anchored, they may be used as reference electrodes for a cardiac mapping operation. Advantageously, the catheter is also configured to preserve at least one perfusion pathway through the coronary sinus from a distal side of the anchor structure to the other. According to the invention, the at least one perfusion pathway is around the exterior of the catheter body and through the interior of the catheter body.

Occasionally, it may be desirable to provide a catheter 130 that completely occludes the coronary sinus. This may be desirable, for example, where catheter 130 is to be employed in conjunction with an ablation procedure. In such contexts, blood flow through the coronary sinus may act as a heat sink, pulling heat away from an ablation site and preventing lesion creation. By occluding the coronary sinus, this heat sink effect may be mitigated.

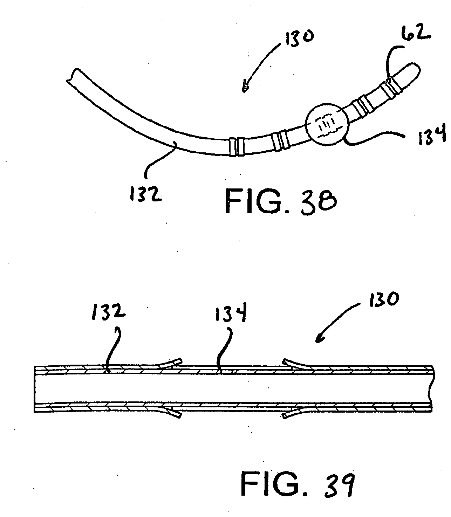

Accordingly, as shown in Figs. 38-40, catheter 130 may include an elongate catheter body 132 and a balloon 134, as well as one or more electrodes (e.g., electrodes 62). When balloon 134 is deflated (Fig. 39), catheter 130 may be introduced into and/or removed from the coronary sinus. When balloon 134 is inflated (Fig. 40), it occludes the coronary sinus. Of course, balloon 134 also serves to anchor catheter 130 relative to the coronary sinus.

Although several examples of the teachings have been described above with a certain degree of particularity, those skilled in the art could make numerous alterations to the disclosed examples without departing from the scope of the invention. For example, though the reference electrode has been described herein as anchored in the coronary sinus, the principles disclosed herein could be employed to anchor the reference electrode in any blood vessel.

Similarly, though the electrode has been described herein as a reference electrode for a localization system, the devices and methods described could also be practiced to position a therapeutic element, such as an RF ablation electrode or other ablation element.

In other examples of the teachings, the catheter body may include roughened surfaces R, as shown in Fig. 37, to increase friction between the catheter body and the tissue surface of the coronary sinus.

As yet another example, a catheter may include multiple balloons positioned along the length of the catheter body, such as shown in Figs. 41 and 42, that permit anchoring of the catheter within the coronary sinus while preserving blood flow through the coronary sinus.

All directional references (e.g., upper, lower, upward, downward, left, right, leftward, rightward, top, bottom, above, below, vertical, horizontal, clockwise, and counterclockwise) are only used for identification purposes to aid the reader's understanding of the present teachings, and do not create limitations, particularly as to the position, orientation, or use of the teachings. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the scope of the invention as defined in the appended claims.

## Claims

1. A catheter (60) for positioning and anchoring an electrode in a coronary sinus for use in a localization or ablation system, the catheter comprising:
an elongate catheter body (64) adapted to be inserted into a coronary sinus, the elongate catheter body (64) including an anchor section (66) having an expandable axial cross-section;
at least one electrode (62) on the catheter body (64); and
an actuation mechanism (70) operably coupled to the anchor section (66) to actuate the anchor section (66) between an undeployed configuration, wherein the expandable axial cross-section of the anchor section (66) is in a collapsed state, and a deployed configuration, wherein the expandable axial cross-section of the anchor section (66) is in an expanded state,
wherein, when the anchor section (66) is in the deployed configuration, the expandable axial cross-section engages a tissue surface of the coronary sinus to inhibit movement between the catheter body (64) and the coronary sinus without completely occluding the coronary sinus, and wherein the catheter body includes at least one perfusion passage through the interior of the catheter body having a first opening positioned distally of the anchor section and a second opening positioned proximally of the anchor section to preserve a perfusion pathway through the coronary sinus.

2. The catheter (60) according to claim 1, wherein the at least one electrode (62) is positioned distally of the anchor section (66) or is positioned proximally of the anchor section (66) or is positioned on the anchor section (66).

3. The catheter (60) according to any one of the preceding claims, wherein the actuation mechanism comprises a tension member (70), wherein placing the tension member (70) in tension causes the anchor section to assume the deployed configuration.

4. The catheter (60) according to any one of the preceding claims, wherein the anchor section (66) comprises at least one expandable member mounted to an outer surface of the catheter body (64).

5. The catheter according to claim 4, wherein the at least one expandable member comprises at least one balloon (72) or at least one wire basket (78).

6. The catheter (60) according to any one of claims 1 to 5, wherein the second opening is formed in a sidewall of the catheter body.

## Patentansprüche

1. Ein Katheter (60) zum Positionieren und Verankern einer Elektrode in einem Koronarsinus zur Verwendung in einem Lokalisierungs- oder Ablationssystem, mit
einem länglichen Katheterkörper (64), der dazu angepasst ist, in einem Koronarsinus eingeführt zu werden, bei dem der längliche Katheterkörper (64) einen Verankerungsabschnitt (66) aufweist, der einen aufweiterbaren axialen Querschnitt aufweist,
zumindest einer Elektrode (62) auf dem Katheterkörper (64), und
einem Betätigungsmechanismus (70), der operativ mit dem Verankerungsabschnitt (66) zum Betätigen des Verankerungsabschnittes (66) zwischen einer nicht entfalteten Konfiguration, bei welcher der aufweiterbare axiale Querschnitt des Verankerungsabschnittes (66) in einem zusammengeklappten Zustand ist, und einer entfalteten Konfiguration, gekoppelt ist, bei dem der aufweiterbare axiale Querschnitt des Verankerungsabschnittes (66) in einem aufgeweiteten Zustand ist,
bei dem, wenn der Verankerungsabschnitt (66) in der entfalteten Konfiguration ist, der aufweiterbare axiale Querschnitt mit einer Gewebeoberfläche des Koronarsinus in Eingriff steht, um eine Bewegung zwischen Katheterkörper (64) und dem Koronarsinus zu verhindern, ohne den Koronarsinus vollständig zu verschließen, und bei dem der Katheterkörper zumindest eine Perfusionspassage durch das Innere des Katheterkörpers aufweist, die eine erste Öffnung, die distal des Verankerungsabschnittes positioniert ist, und eine zweite Öffnung aufweist, die proximal des Verankerungsabschnittes positioniert ist, um eine Perfusionspassage durch den Koronarsinus zu bewahren.

2. Katheter (60) nach Anspruch 1, bei dem die zumindest eine Elektrode (62) distal des Verankerungsabschnittes (66) positioniert ist, oder proximal des Verankerungsabschnittes (66) positioniert ist oder an dem Verankerungsabschnitt (66) positioniert ist.

3. Katheter (60) nach einem der vorhergehenden Ansprüche, bei dem der Betätigungsmechanismus ein Zugbauteil (70) aufweist, bei dem Platzieren des Zugbauteils (70) unter Zug bewirkt, dass der Verankerungsabschnitt die entfaltete Konfiguration einnimmt.

4. Katheter (60) nach einem der vorhergehenden Ansprüche, bei dem der Verankerungsabschnitt (66) zumindest ein aufweiterbares Bauteil aufweist, das auf einer äußeren Oberfläche des Katheterkörpers (64) montiert ist.

5. Katheter nach Anspruch 4, bei dem das zumindest eine aufweiterbare Bauteil zumindest einen Ballon (72) oder zumindest einen Drahtkorb (78) aufweist.

6. Katheter (60) nach der Ansprüche 1 bis 5, bei dem die zweite Öffnung in einer Seitenwand des Katheterkörpers ausgebildet ist.

## Revendications

1. Cathéter (60) pour positionner et fixer une électrode dans un sinus coronaire pour une utilisation dans un système de localisation ou d'ablation, le cathéter comprenant :
un corps de cathéter allongé (64) adapté pour être inséré dans un sinus coronaire, le corps de cathéter allongé (64) comprenant un segment de fixation (66) ayant une coupe transversale axiale dilatable ;
au moins une électrode (62) sur le corps de cathéter (64) ; et
un mécanisme d'actionnement (70) couplé de manière opérationnelle au segment de fixation (66) pour actionner le segment de fixation (66) entre une configuration non déployée, où la coupe transversale axiale dilatable du segment de fixation (66) est dans un état affaissé, et une configuration déployée, où la coupe transversale axiale dilatable du segment de fixation (66) est dans un état dilaté,
dans lequel, lorsque le segment de fixation (66) est dans la configuration déployée, la coupe transversale axiale dilatable vient en prise avec la surface de tissu du sinus coronaire pour empêcher tout mouvement entre le corps de cathéter (64) et le sinus coronaire sans obstruer complètement le sinus coronaire, et dans lequel le corps de cathéter comprend au moins un passage de perfusion à travers l'intérieur du corps de cathéter ayant une première ouverture positionnée de manière distale par rapport au segment de fixation et une seconde ouverture positionnée de manière proximale par rapport au segment de fixation pour préserver un passage de perfusion à travers le sinus coronaire.

2. Cathéter (60) selon la revendication 1, dans lequel ladite au moins une électrode (62) est positionnée de manière distale par rapport au segment de fixation (66) ou est positionnée de manière proximale par rapport au segment de fixation (66) ou est positionnée sur le segment de fixation (66).

3. Cathéter (60) selon l'une quelconque des revendications précédentes, dans lequel le mécanisme d'actionnement comprend un élément de tension (70), dans lequel le fait de placer l'élément de tension (70) en tension amène le segment de fixation à prendre la configuration déployée.

4. Cathéter (60) selon l'une quelconque des revendications précédentes, dans lequel le segment de fixation (66) comprend au moins un élément dilatable monté sur une surface extérieure du corps de cathéter (64).

5. Cathéter selon la revendication 4, dans lequel ledit au moins un élément dilatable comprend au moins un ballon (72) ou au moins un panier en fil métallique (78).

6. Cathéter (60) selon l'une quelconque des revendications 1 à 5, dans lequel la seconde ouverture est formée dans une paroi latérale du corps de cathéter.
